(19) Europäisches Patentamt
European Patent Office
Office européen des brevets

(11) EP 1 886 695 A1

(12) EUROPEAN PATENT APPLICATION

(43) Date of publication:
**13.02.2008 Bulletin 2008/07**

(21) Application number: **06116145.1**

(22) Date of filing: **27.06.2006**

(51) Int Cl.:
***A61K 45/06*** *(2006.01)* ***A61P 5/42*** *(2006.01)*
***A61P 5/46*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Speedel Experimenta AG**
**4123 Allschwil (CH)**

(72) Inventor: **Schumacher, Christoph**
**4123 Allschwil (CH)**

(74) Representative: **Maué, Paul Georg**
**Solvias AG**
**Patents**
**Erlenstrasse 1**
**4058 Basel (CH)**

(54) **Pharmaceutical combination of an aldosterone synthase inhibitor and a glucocorticoid receptor antagonist or a cortisol synthesis inhibitor or a corticotropin releasing factor antagonist**

(57) The invention relates to a pharmaceutical combination comprising (a) an aldosterone synthase inhibitor or a pharmaceutically acceptable salt thereof, and (b) a glucocorticoid receptor antagonist or a cortisol synthesis inhibitor or a cortisol re-synthesis inhibitor or a corticotrophin-releasing hormone receptor antagonist or combinations thereof or in each case a pharmaceutically acceptable salt thereof. Said composition is useful for the manufacture of a medicament, in particular for the manufacture of a medicament for the prevention of, delay of progression of treatment of a disease or condition characterized by the metabolic syndrome.

EP 1 886 695 A1

## Description

### FIELD OF THE INVENTION

The invention relates to pharmaceutical compositions and methods for achieving a therapeutic effect including, but not limited to, the treatment of the metabolic syndrome, including obesity, insulin resistance, hypertension, dyslipidemia and atherosclerosis, in an animal, preferably a mammal including a human subject or a companion animal, using (i) an aldosterone synthase inhibitor or a pharmaceutically acceptable salt thereof in combination with (ii) a glucocorticoid receptor antagonist or a pharmaceutically acceptable salt thereof and/or (iii) a cortisol synthesis inhibitor or a pharmaceutically acceptable salt thereof, (iv) and/or a cortisol re-synthesis inhibitor or a pharmaceutically acceptable salt thereof, (v) and/or a corticotrophin-releasing hormone receptor antagonist or a pharmaceutically acceptable salt thereof and (vi) a pharmaceutically acceptable carrier.

### BACKGROUND OF THE INVENTION

An aggregate of signs and symptoms that constitute together the picture of a disease is commonly named a syndrome. The metabolic syndrome is often defined as a state of metabolic dysregulation characterized by insulin resistance and a predisposition to type 2 diabetes, central and visceral obesity, hypertension and dyslipidemia. Thus, the metabolic syndrome is associated with a marked increased incidence of coronary, cerebral and peripheral artery disease. A combination of overnutrition, physical inactivity, endocrine imbalance as well as genetic and environmental factors interacts to produce a state of metabolic dysregulation that leads to obesity, insulin resistance and hypertension.

An endocrine imbalance underlying the state of metabolic dysregulation can be mediated by the adrenal gland steroid hormones cortisol and aldosterone. Disorders of the adrenal cortex and medulla can result in glucose intolerance or overt diabetes as well as in water retention and hypertension. Cushing's syndrome, characterized by excessive secretion of glucocorticoids, impairs glucose tolerance primarily by causing insulin resistance and enhancing hepatic glucose production. On the other hand, phaeochromocytoma and hyperaldosteronism, via the respective actions of catecholamines and hypokalemia on the pancreatic beta-cell, impair glucose tolerance primarily by inhibiting insulin release. In addition, plasma aldosterone levels determine vascular stiffness, regulate the salt balance and thus blood pressure.

The primary biological function of the glucocorticoid cortisol is to regulate the production and the availability of carbohydrates for the brain and other metabolically active tissues. Increased cortisol production and secretion is a normal physiological response to stress and leads to the essential mobilization of fats, proteins and carbohydrates to meet an increased demand for energy by the body. Glucocorticoids are potent antagonists of insulin and when in excess can promote insulin resistance and obesity. Chronically excessive cortisol release describes the condition of Cushing's syndrome. Cushing syndrome may be produced on one hand by hypersynthesis of cortisol, which may be generated by an adrenocortical tumor, or be produced on the other hand as the consequence of excessive stimulation of the adrenal cortex by adrenocorticotropic hormone (ACTH) whose secretion is mainly controlled by the hypothalamic corticotrophin-releasing hormone (CRH). The first form is referred to as primary hypercortisolism, and the second form as secondary hypercortisolism. An excessive and persistent cortisol secretion may also accompany a stress response, which may lead to depression, hyperglycemia and to suppression of the immune system. Thus, metabolic and Cushing's syndromes share many features, suggesting that abnormalities of glucocorticoid hormone action may contribute to the pathogenesis by promoting lipolysis and triglyceride storage, inducing gluconeogenesis, hypertension and fat cell differentiation.

Aldosterone regulates electrolyte excretion and intravascular volume mainly through its effects on the distal tubules and cortical collecting ducts of the kidney by increasing sodium (Na+) reabsorption and potassium (K+) excretion. The state of excessive aldosterone secretion may lead to sodium and water retention, high blood pressure, hypokalemia, alkalosis, muscle weakness, polyuria, edemas, vasculitits, increased collagen formation, vascular remodeling, tissue fibrosis and endothelial dysfunction. Recent clinical evaluations indicate that primary hyperaldosteronism is common in subjects with resistant hypertension. Resistant hypertension has been conventionally defined as persistently elevated blood pressure in spite of use of three or more antihypertensive agents of different classes, one of which is a diuretic.

Cortisol and aldosterone exert most of their biological effects by binding to their respective intracellular nuclear receptors. In the hormone-bound state, these receptors specifically bind to and modulate the activity of target gene promoters thus eleciting hormone specific genomic responses. The glucocorticoid receptor is ubiquitously expressed and regulates, either directly or indirectly, target genes involved in glucose homeostasis and cell differentiation. The mineralcorticoid receptor is found in epithelial tissues (e.g. kidney, gastrointestinal tract, salivary and sweat glands) as well as in non-epithelial cells (heart, hippocampus, vasculature, mammary gland or leukocytes) and regulates genes involved in salt and tissue homeostasis. Recently, also non-genomic responses to these steroids have been postulated.

Cortisol is a steroidal hormone which is synthesized almost exclusively in the zona fasciculata of the adrenal cortex by the cytochrome P450 enzyme 11-β-hydroxylase (CYP11B1). Cortisol production is controlled by ACTH and

a negative feedback loop via the hypothalamic-pituitary-adrenal axis. The main regulators of intracellular glucocorticoid levels are 11β-hydroxysteroid dehydrogenase (HSD) enzymes. 11β-HSD type 1 is an NADP(H)-dependent enzyme that acts primarily as a reductase, converting the inactive 11-keto metabolites cortisone (in humans) or 11-dehydrocorticosterone (in rodents) into the active glucocorticoids cortisol or corticosterone, respectively. 11β-HSD type 1 is expressed in most tissue types and potentiates the action of endogenous glucocortocids by increasing their local concentration due to re-synthesis. 11β-HSD type 2 is an NAD(H)-dependent enzyme that catalyzes the reverse reaction, oxidizing active glucocorticoids to their inactive 11-keto forms. Although 11β-HSD type 1 is widely expressed, 11β-HSD type 2 expression is limited to tissues that express the mineralocorticoid receptor. By inactivating cortisol, 11β-HSD type 2 prevents it from binding to the mineralcorticoid receptor, thus conferring aldosterone specificity on the receptor.

Against this background, it has been reasoned that subtle abnormalities of steroid biosynthesis or metabolism may contribute to the pathophysiology of the metabolic syndrome. Four pharmacological strategies have been proposed to block excessive cortisol actions: A) the administration of a glucocorticoid receptor antagonist; B) the application of a cortisol synthesis inhibitor, C) the development of a cortisol re-synthesis inhibitor by blocking 11β-hydroxysteroid dehydrogenase type I and D) the use of a corticotrophin-releasing hormone receptor antagonist.

A) Steroidal glucocorticoid receptor antagonist had been derived from the glucocorticoid scaffold. For instance, RU 38486 (mifepristone, 11 β-(4-dimethylaminophenyl)-17β-hydroxy-17α-(1-propylynyl)estra-4,9-dien-3-one), formula (I) is described as an unselective glucocorticoid and progesterone receptor antagonist whereas the derivative RU-43044, formula (II) is reported as a selective glucocorticoid receptor antagonist. Selective, nonsteroidal glucocorticoid receptor antagonists have been derived from RU 38486 and for instance described by Morgan et al. (2002) in J. Med. Chem. 45, 2417-2424, as CP-394531, formula (III) and CP-409069, formula (IV) Other nonsteroidal glucocorticoid receptor antagonist compounds are described for example in following patents and patent applications: US 6,380,223 B1, US 6,436,986 B1, US 6,468,975 B1, US 2002/0147336 A1, US 2002/0107235 A1, US 2004/0014741 A1, US 2004/0176595 A1, WO 2004/009017 A2, WO 2004/110385 A2, WO 2004/111015 A1, US 2004/0266758 A1, US 2004/0266831 A1, WO 2001/16128 A1.

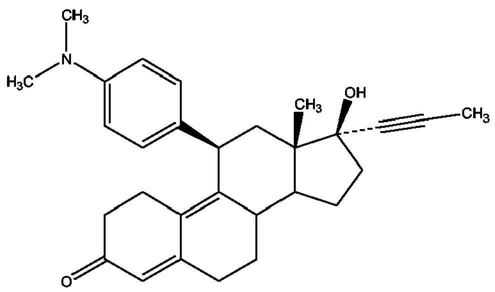

RU-38486 (I)

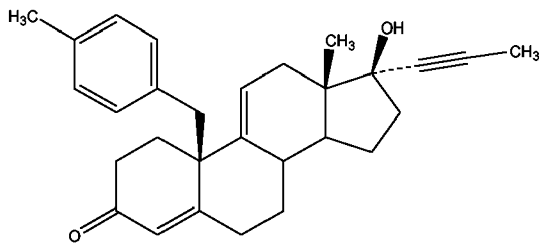

RU-43044 (II)

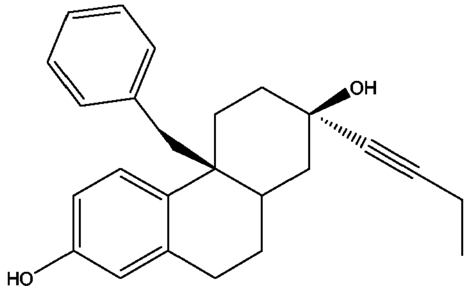

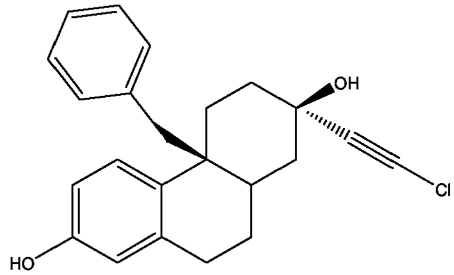

CP-409069 (III)

CP-394531 (IV)

B) Cortisol synthesis inhibitory properties have been ascribed to several drugs. For instance, ketoconazol, formula (V) was initially developed as an anti-fungal therapy. The drug inhibits unselectively the synthesis corticosteroids and at higher doses the synthesis of testosterone as observed by Pont et al., (1982) Ann. Intern. Med 97, 370-372; Engelhardt et al. (1983) Klin. Wochenschr. 61, 373-375; Van Tyle (1984) Pharmacotherapy 4, 343-373. Recently, the use of ketoconazole in cardiovascular and metabolic diseases has been claimed by e.g. US 6,274582 B1, US 6,642,236 B1, US 2006/0014758 A1. Aminogluthetimide, formula (VI) inhibits side-chain cleavage to produce medical adrenalectomy and furthermore several steroidogenic hydroxylation steps as described by Schteingart and Conn (1967) J. Clin. Endocrinol. Metab. 27, 1657-1666; Wipple et al. Steroids (1981) 37, 673-679; Lambert et al. (1984) Mol. Cell. Endocrinol 37, 115-120;. Therefore, aminogluthetimide induces adrenal insufficiency of both, glucocorticoids and mineralocorticoids in patients. Metyrapone, formula (VII) is an unselective 11 β-hydroxylase inhibitor and also used as a diagnostic tool for adrenal function. Trilostane, formula (VIII) is a steroidal 3β-hydroxysteroid dehydrogenase / delta 5-4 isomerase inhibitor in the adrenal cortex. The administration of trilostane results in the inhibition of the synthesis of both, mineralocorticoids and glucocorticods. A similar profile had been observed with CGS-16949A as noted by Lamberts et al. (1989) J. Clin. Endocrinol. Metab. 69, 896-901. Other drugs shown to inhibited cortisol output such as etomidate, epostane, thiopentone, ketotrilostane were described by Lambert et al. (1986) Ann. Clin. Biochem. 23, 225-229. Finally, mitotane, formula (IX) is a chemotherapeutic agent which is cytotoxic to the adrenal gland as studied by Touitou et al. (1979) J. Endocrinol. 82, 87-94. Several other analogous compounds were identified as 11-β-hydroxylase inhibitors such as 4-phenylimidazole, 1-benzylimidazole, 17-β-ureido-1,4-androstadien-3-one, SU-8000, 4-methyl-aminoglutethimide and 20-α-hydroxycholesterol as diescribed by Whipple et al. (1981) Steroids 37, 673-679. Recently, Ulmschneider et al. (2005) J. Med. Chem. 48, 1563-1575, described selective 11-β-hydroxylase (CYP11 B1) inhibitors that are derived from formula (X).

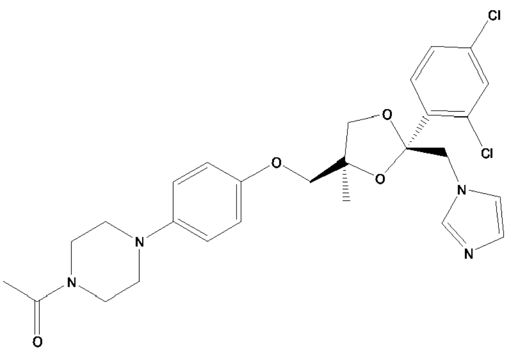

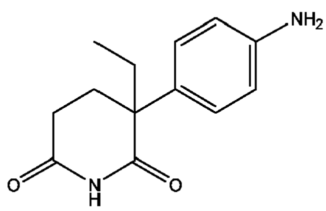

Ketoconazole (V)

Aminogluthetimide (VI)

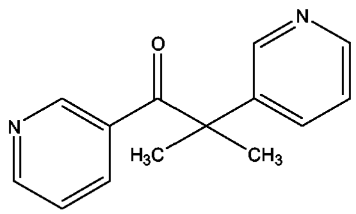

Metyrapone (VII)

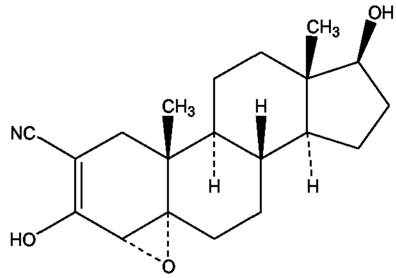

Trilostane (VIII)

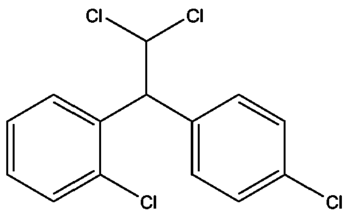

Mitotane (IX)

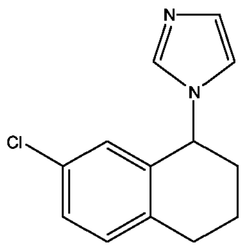

HB6 (X)

C) The selective inhibition of cortisol re-synthesis by blockade of 11β-hydroxysteroid dehydrogenase type I to prevent the conversion of inactive glucocorticoid metabolites into active glucocorticoids in metabolically active tissues is a novel approach that is being investigated. The potential to inhibit 11β-hydroxysteroid dehydrogenase type I has been shown with liquorice extracts, glycyrrhetinic acid and carbenoxolone, formula (XI) as described by Andrews et al. (2003) J. Clin. Endocrinol. Metab. 88, 285-291; Li eta I. (2004) 53, 600-606. Chenodeoxycholic acid and metyrapone have also been described as enzyme inhibitors by Morris et al. (2004) 53, 811-816; Sampath-Kumar et al. (1997) 62, 195-199. Other structures such as perhydoquinolylbenzamides and flavanones have been characterized as 11β-hydroxysteroid dehydrogenase type I inhibitors by Coppola et al. (2005) Metabolism 48, 6696-6712 and Schweizer at al. (2003) Mol. Cell. Endocrinol. 212, 41-49. Compound 544, formula (XII) is described in the literature as a potent and enzyme selective inhibitor.

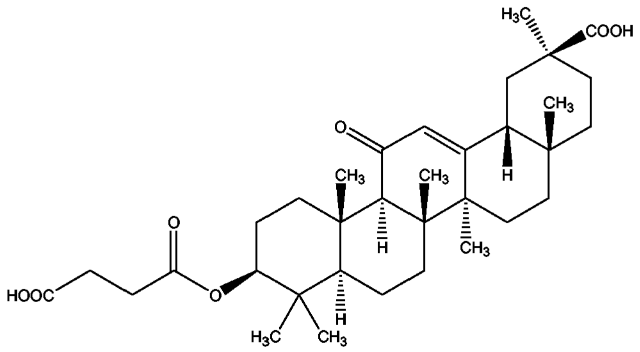

Carbenoxolone (XI)

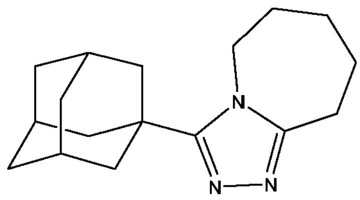

Compound 544 (XII)

D) Antagonists of the corticotrophin-releasing hormone receptors have been identified and described, for example by Chen et al. (1997) in J. Med. Chem. 40, 1749-1754 reporting the discovery of CP-154526, formula (XIII) or by Chen et al. (2004) in J. Med. Chem. 47, 4787-4798 reporting the identification of compound 26h, formula (XIV). Furthermore, patents and patent applications report novel corticotrophin-releasing hormone receptor antagonists such as, for example US 6492520 B1, US 6245769 B1, US 5880135, EP 0691128 B1, WO 99/11643, US 2001/0025042 A1, US 2003/0229091 A1, WO 02/089814 A1.

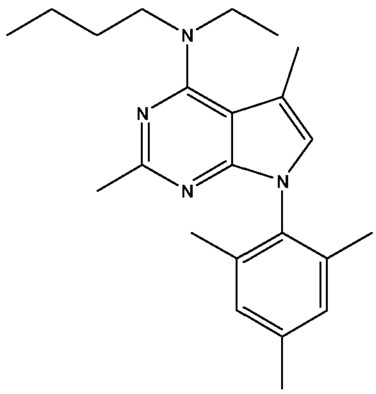

CP-154526 (XIII)

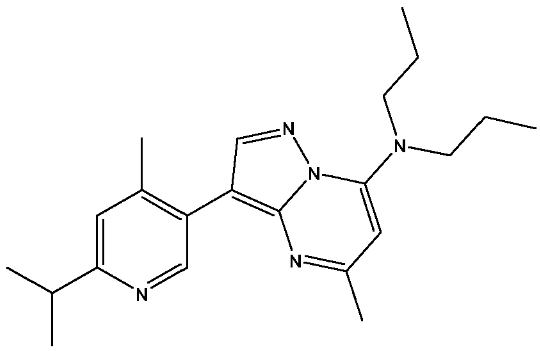

Compound 26h (XIV)

Aldosterone is a steroidal hormone that is mainly synthesized in the zona glomerulosa of the adrenal cortex by the enzyme aldosterone synthase (CYP11B2). The aldosterone synthase mediates the rate limiting conversion of 18-hydroxy-corticosterone into aldosterone. The synthesis and secretion of aldosterone is controlled by angiotensin II (Ang II), potassium (K+) excess and sodium (Na+) deficiency, as well as by adrenocorticotropic hormone. After the development of mineralocorticoid receptor blockers, a new pharmacological approach has been described to reduce excessive aldosterone actions. The approach consists in inhibiting aldosterone synthase (CYP11 B2), the rate-limiting and final enzymatic step for aldosterone synthesis.

The imidazoles are a class of synthetic compounds that inhibit various cytochrome P450-mediated steroid hydroxylations. Certain imidazole derivatives have been described that exert inhibitory effects on aldosterone synthase (cf US 5057521, JP 97-071586, WO 2001/76574 A2, WO 2004/046145 A1, WO 2004/014914 A1, WO 2005/118581 A1, WO 2005/118557 A2, WO 2005/118541 A2. Fadrozole of formula (XV) (cf. US patents 4617307 and 4889861), for example, is an imidazole that has been described by Demers et al. (1990) in the J. Clin. Endocrinol. Metab. 70, 1162-1166, to block specifically aromatase (CYP19) at daily dosages of 1-2 mg in postmenopausal patients with metastatic breast cancer yet at doses of 16 mg daily produced significant suppression of both basal and ACTH-stimulated aldosterone production. This effect was accompanied by a significant rise in the blood 18-hydroxy-corticosterone/aldosterone ratio

consistent with an aldosterone synthase inhibition.

Recently, the fadrozole enantiomer FAD286, (+)-(5R)-4-(5,6,7,8-tetrahydro-imidazo[1,5-a]pyridine-5-yl)benzonitrile hydrochloride of formula (XVI) (cf. WO 2001/76574 A2), has been reported by Fiebeler et al. (2005) Circulation 111, 3087-3094 to inhibit potently aldosterone synthase..

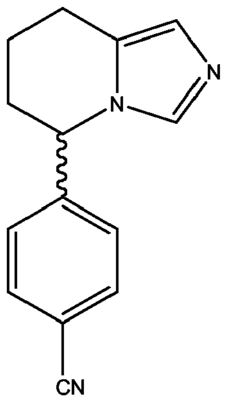

Fadrozole (XV)

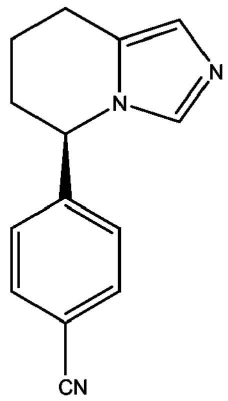

FAD286 (XVI)

DETAILED DESCRIPTION OF THE INVENTION

The combined administration of an aldosterone synthase inhibitor together with an cortisol-suppressing agent, be it in form of a glucocorticoid receptor antagonist or a cortisol synthesis inhibitor or a cortisol re-synthesis inhibitor or a corticotrophin-releasing hormone receptor antagonist or a combination of said antagonists and inhibitors, in conditions characterized by hyperaldosteronism and hypercortisolism such as described for the metabolic syndrome offer the following benefits:

(i) Treatment of multiple risk factors
(ii) Improved therapeutic profile
(iii) Improved safety profile

The term "benefit" as used herein means that the effect achieved with the methods and compositions of the present invention is greater than the sum of the effects that result from methods and compositions comprising the active ingredients of this invention separately.

Thus, an object of the instant invention is a pharmaceutical composition comprising

(a) an aldosterone synthase inhibitor or a pharmaceutically acceptable salt thereof,
(b) - a glucocorticoid receptor antagonist or

- a cortisol synthesis inhibitor or
- a cortisol re-synthesis inhibitor or
- a corticotrophin-releasing hormone receptor blocker or
- any combination of a glucocorticoid receptor antagonist, a cortisol synthesis inhibitor, a cortisol re-synthesis inhibitor and a corticotrophin-releasing hormone receptor blocker,

and in each case a pharmaceutically acceptable salt thereof, and
(c) a pharmaceutically acceptable carrier.

Above composition also relates to combinations of at least two different active compounds within one of the groups of cortisol suppressing agents, for example, two or more glucocorticoid receptor antagonists.

A person skilled in the pertinent art is fully enabled to select a relevant and standard animal test model to show the hereinafter indicated therapeutic indications and beneficial effects. For example, the beneficial effects can be demonstrated in a test system as follows:

Experimental Method

The inhibition of the enzymes 11 β-hydroxylase (CYP11B1) and aldosterone synthase (CYP11B2) by compounds such as described by formula (V -X) and formula (XIII) may be tested by following *in vitro* assay. The human cell line NCI-H295R that was originally isolated from an adrenal carcinoma is extensively described in the literature for its stimulus-dependent steroid hormone secretion. The cells have the physiological characteristics of zonally undifferentiated human fetal adrenal cells, with the ability to produce the steroid hormones of each of the three phenotypically distinct zones found in the adult adrenal cortex.

The NCI-295R cells (American Type Culture Collection, ATCC, Rockville, MD, USA) are cultured in Dulbecco's Modified Eagle'Ham F-12 medium (DME/F12) that is supplemented with Ultroser SF serum (Soprachem, Cergy-Saint-Christophe, France) as well as insulin, transferring, selenit (I-T-S, Becton Dickinson Biosciences, Franklin Lakes, NJ, USA) and antibiotics in 75 $cm^2$ cell culture flasks at a temperature of 37 °C and a 95% air/5% CO2 humidified atmosphere. The cells are subsequently transferred in a 24-well plate and seeded in presence of DME/F12 medium that is supplemented with 0.1% bovine serum albumin instead of Ultroser SF serum. The experiment is initiated by incubating the cells for 72 h in DME/F12 medium supplemented with 0.1% bovine serum albumin and test compounds in presence or absence of cell stimulatory agents. The test compound or the mixture of test compounds is added in a concentration range of 0.2 nanomolar to 20 micromolar. As cell-stimulatory agents may serve angiotensin-II (at 10 or 100 nanomolar concentration), potassium ions (at 16 millimolar), forskolin (at 10 micromolar) or a combination of two agents. The cellular secretion of aldosterone, cortisol, corticosterone and estradiol/estrone into the cell culture medium can be quantitatively assessed with commercially available immuno-assays and specific monoclonal antibodies according to the manufacturer's instructions.

The degree of secretion of a selective steroid is used as a measure of enzyme activity, respectively enzyme inhibition in presence of absence of a test compound. The dose-dependent enzyme inhibitory activity of a compound is reflected in an inhibition curve that is characterized by an IC50 value. The IC50 values for active test compounds are generated by simple linear regression analysis to establish inhibition curves without data weighing. The inhibition curve is generated by fitting a 4-parameter logistic function to the raw data of the samples using the least squares approach. The function is described as follows:

$$Y = (d-a) / ((1 + (x/c)^{-b})) + a$$

with:

a = minimum
b = slope
c= IC50
d = maximum
x = inhibitor concentrations

The combinations of the present invention of an aldosterone synthase inhibitor and a cortisol synthesis inhibitor show inhibitory effects with minimal concentrations of about $10^{-3}$ to about $10^{-10}$ mol/l.

The cortisol- and aldosterone-reducing effects of combinations of the present invention can be tested *in vivo* by the following protocol: Adult male Sprague Dawley rats, weighing between 125 and 150 grams are kept individually housed under the usual conditions of light and temperature. At 16:00 h on the first day of the experiment, the animals receive a subcutaneous injection of the depot ACTH in a dose of 1.0 mg/kg of weight (SYNACTEN-Depot, Novartis, Basel, CH). Pilot studies show that this ACTH dose increased plasma aldosterone and corticosterone significantly by approximately 10-fold and 15-fold, respectively, over a period of up to 18 hours. At 8:00 h in the morning of the second day, the animals, divided into test groups of 5 animals receive administered either water or a test compound in a variable dose range of 0.01 - 10 mg/kg orally by gavage. Subsequently, blood samples are taken in EDTA-treated Eppendorf tubes after various time intervals ranging from 0.5 h to 12 h. Plasma samples are obtained by centrifugation of the blood and can be stored at -20°C. The plasma samples are tested for their steroid content using commercially available steroid-specific radioimmunoassay. The time-dependent reduction of plasma steroid levels upon administration of a test compound is used as measure for pharmacodynamic efficacy and selectivity.

The combinations according to the present invention comprising an aldosterone synthase inhibitor formula (XV-XVI) for example or a pharmaceutically acceptable salt thereof together with a glucocorticoid receptor blocker of formula (I-IV) for example or a pharmaceutically acceptable salt thereof, or a cortisol synthesis inhibitor of formula (V-X), respectively a cortisol re-synthesis inhibitor of formula (XI-XII) or a pharmaceutically acceptable salt thereof, respectively a corticotrophin-releasing hormone receptor antagonist of formula (XIII-XIV) can be administered by various routes of administration but are tested in this example upon oral administration. Each agent can be tested over a wide range of dosages to determine the optimal drug level for each agent in combination to elicit the maximal response. Each study is best performed in which the effects of the combination treatment group are determined at the same time as the individual components are evaluated. Although drug effects may be observed with acute administration (such as day 1), it is preferable to observe metabolic responses to steroid hormone suppression in a chronic setting as shown below in which experiments are done over a two to seven week observation period. The long-term study is of sufficient duration to allow for the full development of compensatory responses to occur and therefore, the observed effect will most likely depict the actual responses of the test system representing sustained or persistent effects.

The obese spontaneously hypertensive rat (SHROB, Koletsky rat) is a unique strain with genetic obesity, hypertriglyceridemia, hyperinsulinemia, renal disease with proteinuria and genetically determined hypertension, all characteristics paralleling the human metabolic syndrome. SHROB rats have exaggerated circadian rhythms and abnormalities in hypothalamic function leading the hypersecretion of corticosterone. Despite multiple metabolic derangements, insulin resistance and hypertension are independent in this model.

Statistical Analysis

The combination therapy can be compared to that of the monotherapy groups by determining the change in plasma steroid parameters and change is single or multiple disease parameters. All values are represented as the group mean ± SEM. Statistical significance is obtained when $p < 0.05$. The AUC values for each of the treatment groups can be compared statistically using a one-way ANOVA followed by the appropriate post-hoc analysis, for example by performing a Tukey's test.

Study design

The experiments are, typically, carried out with 5 treatment groups consisting of:

- Control animals
- SHROB plus vehicle
- SHROB plus aldosterone synthase inhibitor of formula (XV-XVI) at dose 1, 2, 3
- SHROB plus glucocorticoid receptor antagonist of formula (I-IV) at dose 1, 2, 3
- SHROB plus aldosterone synthase inhibitor at dose 1, 2, 3 and glucocorticoid receptor antagonist of formula (I-IV) at dose 1, 2, 3;

It will be appreciated that above defined last and penultimate treatment groups may be composed of more than one cortisol suppressing agent, as contemplated throughout this invention, for instance:

- SHROB plus glucocorticoid receptor antagonist of formula (I-IV) at dose 1, 2, 3 and/or cortisol synthesis inhibitor of formula (V-X) at dose 1, 2, 3 and/or cortisol re-synthesis inhibitor of formula (XI-XII) at dose 1, 2, 3; and/or corticotrophin-releasing hormone receptor antagonist of formula (XIII-XIV) at dose 1, 2, 3
- SHROB plus aldosterone synthase inhibitor at dose 1, 2, 3 and glucocorticoid receptor antagonist of formula (I-IV) at dose 1, 2, 3; and/or cortisol synthesis inhibitor of formula (V-X) at dose 1, 2, 3; and/or cortisol re-synthesis inhibitor of formula (XI-XII) at dose 1, 2, 3; and/or corticotrophin-releasing hormone receptor antagonist of formula (XIII-XIV) at dose 1, 2, 3.

depending on the goals of the study.

The blood pressure of the mice will be monitored continuously via chronically implanted telemetric devices. In order to stimulate the renin angiotensin system an ALZET minipump may be applied in order to release subcutaneously at a constant rate a low dose of angiotensin II. Two hours after the last administration of the test compounds, blood samples will be taken from tail vein and by cardiac puncture, respectively under isoflurane anesthesia. The plasma samples are subjected to blood chemistry. Blood chemistry may determine the concentrations of aldosterone, corticosterone, glucose, and triglycerides. At the end of the study, kidneys and heart of the animals will be collected and subjected to weight and structural analysis.

The dose 1, 2 and 3 are selected in prior pilot experiments using the SHROB rat model to achieve a pharmacological effect of low, intermediate and high potency, respectively. Low is defined as a sub-therapeutic dose with a minimal plasma steroid level change and pharmacodynamic effect. Intermediate is defined as a therapeutic dose with normalizing plasma steroid levels and optimal pharmacodynamic effects. High is defined as a supratherapeutic dose with maximal plasma steroid suppression and pharmacodynamic response. For an aldosterone synthase inhibitor of

formula (XV-XVI) for example, the low dose may range from 0.05 mg/kg/day to 2 mg/kg/day, the intermediate dose from 0.2 mg/kg/day to 8 mg/kg/day and the high dose from 1 mg/kg/day to 40 mg/kg/day. Accordingly, for a glucocorticoid receptor antagonist of formula (I-IV) or a corticotrophin-releasing hormone receptor antagonist of formula (XIII-XIV) for example, the low dose may range from 0.5 mg/kg/day to 10 mg/kg/day, the intermediate dose from 10 mg/kg/day to 100 mg/kg/day and the high dose from 100 mg/kg/day to 500 mg/kg/day. For a cortisol synthesis inhibitor of formula (V-X) or a cortisol re-synthesis inhibitor of formula (XI-XII) for example, the low dose may range from 0.5 mg/kg/day to 20 mg/kg/day, the intermediate dose from 10 mg/kg/day to 50 mg/kg/day and the high dose from 50 mg/kg/day to 200 mg/kg/day.

Plasma and urine analytes

Serum is separated from nonheparinized blood. For urine analysis, the mice are individually housed in metabolic cages and 24 hour urine collected on 1 ml HCl 6 N. The concentrations of the steroids aldosterone, corticosterone, estradiol, dihydroepiandrostendione and dihydrotestosterone are measured by radioimmunoassays. Blood glucose is determined at various time points from tail bleeds using a glucometer. Plasma insulin is assayed using an ELISA kit. Plasma triglyceride and free fatty acid concentrations are measured using an enzymatic colometric test. Protein concentration in the urine is determined by the Coomassie blue G dye-binding method and urinary albumin by an ELISA assay. The concentrations of Na+ and K+ are measured by flame photometry.

Results

The animal studies reveal for an aldosterone-synthase inhibitor a dose-dependent suppression of aldosterone plasma levels and aldosterone-mediated effects on blood pressure as well as cardiac and kidney function. At the supratherapeutic doses a suppression of corticosterone plasma levels is observed. The administration of a glucocorticoid or a corticotrophin-releasing hormone receptor antagonist reveals a dose-dependent suppression of corticosterone-mediated effects on glycemia and triglyceridemia as well as insulin resistance. At the supratherapeutic doses an increase in plasma sexsteroid hormone levels is observed with a glucocorticoid receptor antagonist. The administration of a cortisol synthesis inhibitor or a cortisol re-synthesis inhibitor results in a dose-dependent reduction in plasma glucose and triglyceride concentrations.

The combined administration of an aldosterone synthase inhibitor and a glucocorticoid receptor/corticotrophin-releasing hormone antagonist or cortisol synthesis/re-synthesis inhibitor reveals a therapeutic effect on aldosterone-mediated blood pressure as well as cardiac and kidney function as well as a therapeutic effect on corticosterone-mediated plasma glycemia and triglyceridemia as well as cardiac and kidney function. The individual drugs applied in combination at therapeutic doses each yield a more potent effect on cardiorenal function than applied individually and the effect is not compromised by changes in plasma steroid levels i.e. stress hormones and sex-hormones.

The animal studies indicate that the combined administration of an aldosterone synthase inhibitor and a glucocorticoid receptor antagonist and/or a cortisol synthesis inhibitor and/or a cortisol re-synthesis inhibitor and/or a corticotrophin-releasing hormone receptor antagonist may represent an effective therapy of conditions characterized by an aggregation of multiple diseases as defined for the metabolic syndrome. Accordingly, the invention of combined administration of an aldosterone synthase inhibitor together with a glucocorticoid blocking/suppressing agent relates also to a method for the prevention of, delay of progression of, treatment of a disease or condition selected from the group consisting of adrenal disease, primary aldosteronism, hyperaldosteronism, adrenal incidentalomas, essential hypertension and treatment-resistent hypertension, cushing's syndrome, insulin resistance, impaired glucose tolerance, diabetes mellitus type I and type II, obesity, metabolic syndrome, syndrome X, diabetic and non-diabetic nephropathy, glomerulosclerosis, diabetic retinopathy, macular degeneration, cataracts, diabetic neuropathy, foot, ulcerations, ulcerative colitis, erectile dysfunction, premenstrual syndrome, angina pectoris, congestive heart failure, renal failure, myocardial infarction, survival post-myocardial infarction, coronary heart disease, stroke, vascular restinosis, skin and connective tissue disorders, marfan syndrome, hypertension with increased formation of collagen, fibrosis, remodeling and endothelial dysfunction, atherosclerosis and vascular compliance, diseases and conditions characterized by hyperaldosteronism or relative aldosterone excess along with hypercortisolism or relative cortisol excess.

The methods comprise the administration to a warm-blooded animal, including man, in need thereof a jointly effective amount of a combination of an aldosterone synthase inhibitor and a glucocorticoid receptor antagonist, or a cortisol synthesis inhibitor, or a cortisol re-synthesis inhibitor, or a corticotrophin-releasing hormone receptor antagonist or a combination of said antagonists and inhibitors. The combined administration of an aldosterone synthase inhibitor together with a glucocorticoid receptor antagonist or a cortisol synthesis inhibitor, or a cortisol re-synthesis inhibitor, or a corticotrophin-releasing hormone receptor blocker may represent a better tolerated and therefore safer therapy of conditions characterized by endocrine dysbalance. The benefits when applying the composition of the present invention are that lower doses of the individual drugs to be combined can be used to diminish the incidence of side effects. Lower

doses may also imply that the dosages need to be applied less frequently.

The therapeutically effective amounts of the individual drugs according to the present invention can be administered simultaneously or sequentially in any order, separately or in fixed combination. In a variation thereof, the present invention likewise relates to a "kit-of-parts", for example, in the sense that the components to be combined can be dosed independently or by use of different fixed combinations with distinguished amounts of the components. The parts of the kit of parts can then e.g. be administered simultaneously or chronologically staggered, that is at different time points and with equal or different time intervals for any part of the kit parts. Preferably, the time intervals are chosen such that the effect on the treated disease or condition in the combined use of the parts is larger and better tolerated than the effect that would be obtained by use of only one of the components.

The invention furthermore relates to a commercial package comprising the combination according to the present invention together with instructions for simultaneous, separate or sequential use.

The pharmaceutical preparations comprising the pharmacological active compound or combination of compounds either alone or together with customary pharmaceutical auxiliary substances are for enteral, such as oral, and rectal or parenteral administration to homeotherms. For example, the pharmaceutical preparations may consist from 1 % to 80% of the active compound or combination of compounds. Pharmaceutical preparations for enteral or parenteral and also for ocular administration are, for example, in unit dose forms such as coated tablets, tablets, capsules or suppositories and also ampoules. These are prepared in a manner that is known per se, for example using conventional mixing, granulation, coating, solubilizing or lyophilizing processes. Thus, the pharmaceutical preparations for oral use can be obtained and, if required or necessary, by processing the mixture or granulate into tablets or coated tablet cores after having added auxiliary substances.

The dosage of the active compounds can depend on a variety of factors, such as mode of administration, homeothermic species, age and/or individual condition. Preferred dosages for the active drugs of the pharmaceutical combination according to the present invention are therapeutically effective dosages, especially those inferior or equal to the commercial recommendation of the individual active drug. Normally, in the case of oral administration for a patient of approximately 75 kg in weight, an approximate daily dose for an aldosterone synthase inhibitor as represented by compounds of formula (XV-XVI) may range from 0.2 mg to 200 mg per person per day, respectively from 5 to approximately 1000 mg for a glucocorticoid receptor antagonist as represented by RU-38486 of formula (I), and from 100 to 800 mg for a cortisol synthesis inhibitor as represented by ketoconazole of formula (V) and from 20 to 500 mg for a cortisol re-synthesis inhibitor as represented by carbenoxolone of formula (XI). The daily administration may be divided preferably into 1 to 4 single doses which may, for example be of the same size. Usually children receive about half of the adult dose.

The dose necessary for each individual can be monitored, for example by measuring the serum concentration of the active drugs and or the plasma steroid hormone levels and adjusted to an optimal level.

## Claims

**1.** A pharmaceutical composition comprising

(a) an aldosterone synthase inhibitor or a pharmaceutically acceptable salt thereof,
(b)

- a glucocorticoid receptor antagonist or
- a cortisol synthesis inhibitor or
- a cortisol re-synthesis inhibitor or
- a corticotrophin-releasing hormone receptor blocker or
- any combination of a glucocorticoid receptor antagonist, a cortisol synthesis inhibitor, a cortisol re-synthesis inhibitor and a corticotrophin-releasing hormone receptor blocker

and in each case a pharmaceutically acceptable salt thereof, and
(c) a pharmaceutically acceptable carrier.

**2.** A pharmaceutical composition according to claim 1 wherein said aldosterone synthase inhibitor is fadrozole (compound of formula (XV)), or (+)-(5R)-4-(5,6,7,8-tetrahydro-imidazo[1,5-a]pyridine-5-yl)benzonitrile hydrochloride (compound of formula (XVI)), or in each case a pharmaceutically acceptable salt thereof

**3.** A pharmaceutical composition according to claim 1 or 2 wherein said glucocorticoid receptor antagonist is mifepristone (compound of formula (I)), or a pharmaceutically acceptable salt thereof..

**4.** A pharmaceutical composition according to any of claims 1 to 3 wherein said cortisol synthesis inhibitor is ketoconazole (compound of formula (V)), aminogluthetimide (compound of formula (VI)), metyrapone,(compound of formula (VII)) trilostane (compound of formula (VIII)), mitotane (compound of formula (IX)) or in each case a pharmaceutically acceptable salt thereof.

**5.** A pharmaceutical composition according to claim 1 to 4 wherein said cortisol re-synthesis inhibitor is carbenoxolone (compound of formula (XI)), or a pharmaceutically acceptable salt thereof.

**6.** A pharmaceutical composition according to claim 1 to 4 wherein said corticotrophin-releasing hormone receptor blocker is a compound of formula (XIII) or (XIV),

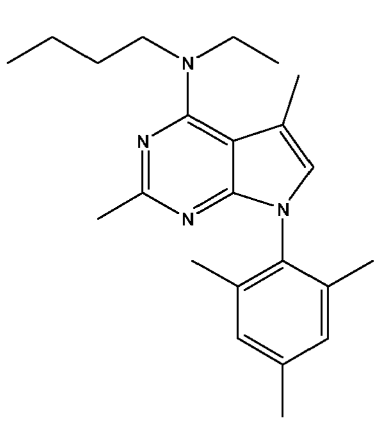

(XIII)

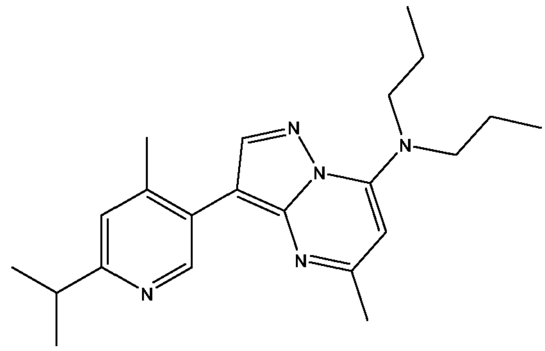

(XIV)

or a pharmaceutically acceptable salt thereof..

**7.** Use of a combination comprising

a. an aldosterone synthase inhibitor or a pharmaceutically acceptable salt thereof, and
b. a glucocorticoid receptor antagonist or a cortisol synthesis inhibitor or a cortisol re-synthesis inhibitor or a corticotrophin-releasing hormone receptor antagonist or combinations thereof or in each case a pharmaceutically acceptable salt thereof

for the manufacture of a medicament, in particular for the manufacture of a medicament for the prevention of, delay of progression of treatment of a disease or condition **characterized by** the metabolic syndrome.

**8.** Use according to claim 7 for the manufacture of a medicament for the prevention of, delay of progression of treatment of a disease or condition **characterized by** hyperaldosteronism or aldosterone excess as well as hypercortisolims or cortisol excess such as adrenal disease, primary aldosteronism, primary hyperaldosteronism, adrenal incidentalomas, essential hypertension and treatment-resistent hypertension, Cushing's syndrome, insulin resistance, impaired glucose tolerance, diabetes mellitus type I and type II, obesity, metabolic syndrome, syndrome X, cushing's syndrome, diabetic and non-diabetic nephropathy, glomerulosclerosis, diabetic retinopathy, macular degeneration, cataracts, diabetic neuropathy, foot, ulcerations, ulcerative colitis, erectile dysfunction, premenstrual syndrome, angina pectoris, congestive heart failure, renal failure, myocardial infarction, survival post-myocardial infarction, coronary heart disease, stroke, vascular restinosis, skin and connective tissue disorders, Marfan syndrome, hypertension with increased formation of collagen, fibrosis, atherosclerosis and vascular compliance, remodeling and endothelial dysfunction.

**9.** A method for the prevention of, delay of progression of, treatment of a disease or condition **characterized by** hyperaldosteronism or aldosterone excess as well as hypercortisolims or cortisol excess such as adrenal disease, primary aldosteronism, primary hyperaldosteronism, adrenal incidentalomas, essential hypertension and treatment-resistent hypertension, Cushing's syndrome, insulin resistance, impaired glucose tolerance, diabetes mellitus type I and type II, obesity, metabolic syndrome, syndrome X, cushing's syndrome, diabetic and non-diabetic nephropathy, glomerulosclerosis, diabetic retinopathy, macular degeneration, cataracts, diabetic neuropathy, foot, ulcerations,

ulcerative colitis, erectile dysfunction, premenstrual syndrome, angina pectoris, congestive heart failure, renal failure, myocardial infarction, survival post-myocardial infarction, coronary heart disease, stroke, vascular restinosis, skin and connective tissue disorders, Marfan syndrome, hypertension with increased formation of collagen, fibrosis, atherosclerosis and vascular compliance, remodeling and endothelial dysfunction **characterized in that** an effective amount of a pharmaceutical composition according to any of claims 1 to 6 is used.

European Patent Office

# EUROPEAN SEARCH REPORT

Application Number
EP 06 11 6145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/118541 A2 (SPEEDEL EXPERIMENTA AG [CH]; HEROLD PETER [CH]; MAH ROBERT [CH]; TSCHI) 15 December 2005 (2005-12-15)<br>* page 8, last paragraph; claims 9,10 *<br>----- | 1-9 | INV.<br>A61K45/06<br>A61P5/42<br>A61P5/46 |
| Y | WO 2006/005726 A2 (SPEEDEL EXPERIMENTA AG [CH]; HEROLD PETER [CH]; MAH ROBERT [CH]; TSCHI) 19 January 2006 (2006-01-19)<br>* page 8, last paragraph; claims 9,10 *<br>----- | 1-9 | |
| Y | WO 2005/118557 A2 (SPEEDEL EXPERIMENTA AG [CH]; HEROLD PETER [CH]; MAH ROBERT [CH]; TSCHI) 15 December 2005 (2005-12-15)<br>* page 7; claims 9,10 *<br>----- | 1-9 | |
| Y | MELLIN VIRGINIE ET AL: "Long-term aldosterone synthase inhibition improves cardiac function and myocardial structure in chronic heart failure"<br>CIRCULATION,<br>vol. 110, no. 17, Suppl. S,<br>October 2004 (2004-10), pages 513-514,<br>XP009075442<br>& 77TH SCIENTIFIC MEETING OF THE AMERICAN-HEART-ASSOCIATION; NEW ORLEANS, LA, USA; NOVEMBER 07 -10, 2004<br>ISSN: 0009-7322<br>* abstract *<br>-----<br>-/-- | 1-9 | TECHNICAL FIELDS SEARCHED (IPC)<br>A61K<br>A61P |

The present search report has been drawn up for all claims

1

| Place of search | Date of completion of the search | Examiner |
|---|---|---|
| Munich | 1 December 2006 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 11 6145

| Category | Citation of document with indication, where appropriate, of relevant passages | Relevant to claim | CLASSIFICATION OF THE APPLICATION (IPC) |
|---|---|---|---|
| Y | VOETS M ET AL: "Synthesis and evaluation of heteroaryl-substituted dihydronaphthalenes and indenes: potent and selective inhibitors of aldosterone synthase (CYP11B2) for the treatment of congestive heart failure and myocardial fibrosis" JOURNAL OF MEDICINAL CHEMISTRY, AMERICAN CHEMICAL SOCIETY. WASHINGTON, US, vol. 49, no. 7, 8 March 2006 (2006-03-08), pages 2222-2231, XP002386733 ISSN: 0022-2623 * page 2222 * | 1-9 | |
| Y | WO 01/52833 A1 (CORTENDO AB [SE]; GARDNER REBECCA [GB]; MAERIN PER [SE]; LANDH TOMAS [) 26 July 2001 (2001-07-26) * page 4, last paragraph - page 6, line 17; claims 2,28,29,32 * | 1-4,7-9 | |
| Y | CHU JAMES W ET AL: "Successful long-term treatment of refractory Cushing's disease with high-dose mifepristone (RU 486)" JOURNAL OF CLINICAL ENDOCRINOLOGY AND METABOLISM, vol. 86, no. 8, August 2001 (2001-08), pages 3568-3573, XP002410091 ISSN: 0021-972X * abstract * | 1-3,7-9 | TECHNICAL FIELDS SEARCHED (IPC) |
| Y | EP 0 903 146 A (APPLIED RESEARCH SYSTEMS [AN]) 24 March 1999 (1999-03-24) * claims 3,4 * | 1-3,7-9 | |
| Y | WO 96/04912 A (METABOLIC SYNDROME I GBG AB [SE]; MAARIN PER [SE]) 22 February 1996 (1996-02-22) * page 1, last paragraph; claims * | 1,2,4, 7-9 | |
| | -/-- | | |

The present search report has been drawn up for all claims

1

| Place of search | Date of completion of the search | Examiner |
|---|---|---|
| Munich | 1 December 2006 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 06 11 6145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 097 709 A2 (PFIZER PROD INC [US]) 9 May 2001 (2001-05-09) * paragraphs [0002], [0021] * | 1,2,6-9 | |
| A | BAEHR V ET AL: "THE METABOLIC SYNDROME X AND PERIPHERAL CORTISOL SYNTHESIS" EXPERIMENTAL AND CLINICAL ENDOCRINOLOGY AND DIABETES, JOHANN AMBROSIUS BARTH, DE, vol. 110, no. 7, 2002, pages 313-318, XP008015667 ISSN: 0947-7349 * the whole document * | 1-9 | |
| A | FALLO F ET AL: "Prevalence and characteristics of the metabolic syndrome in primary aldosteronism" JOURNAL OF CLINICAL ENDOCRINOLOGY AND METABOLISM 2006 UNITED STATES, vol. 91, no. 2, February 2006 (2006-02), pages 454-459, XP009075525 ISSN: 0021-972X 0021-972X * the whole document * | 1-9 | TECHNICAL FIELDS SEARCHED (IPC) |

The present search report has been drawn up for all claims

1

| Place of search | Date of completion of the search | Examiner |
|---|---|---|
| Munich | 1 December 2006 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 11 6145

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2006

| Patent document cited in search report | Publication date | Patent family member(s) | Publication date |
|---|---|---|---|
| WO 2005118541 A2 | 15-12-2005 | AR 050251 A1 | 11-10-2006 |
| WO 2006005726 A2 | 19-01-2006 | AR 049711 A1 | 30-08-2006 |
| WO 2005118557 A2 | 15-12-2005 | AR 049388 A1 | 26-07-2006 |
| WO 0152833 A1 | 26-07-2001 | US 2002055512 A1 | 09-05-2002 |
| EP 0903146 A | 24-03-1999 | AT 215373 T | 15-04-2002 |
| | | AU 738522 B2 | 20-09-2001 |
| | | AU 1334199 A | 12-04-1999 |
| | | CA 2303817 A1 | 01-04-1999 |
| | | DE 69804659 D1 | 08-05-2002 |
| | | DE 69804659 T2 | 02-10-2002 |
| | | DK 1033990 T3 | 08-07-2002 |
| | | WO 9915181 A1 | 01-04-1999 |
| | | ES 2172937 T3 | 01-10-2002 |
| | | IL 135232 A | 17-05-2005 |
| | | PT 1033990 T | 31-07-2002 |
| | | US 6303591 B1 | 16-10-2001 |
| | | ZA 9808674 A | 28-06-1999 |
| WO 9604912 A | 22-02-1996 | US 5849740 A | 15-12-1998 |
| EP 1097709 A2 | 09-05-2001 | AU 776724 B2 | 16-09-2004 |
| | | AU 6669500 A | 03-05-2001 |
| | | CA 2325069 A1 | 29-04-2001 |
| | | HU 0004194 A2 | 28-12-2001 |
| | | NZ 507825 A | 26-11-2004 |
| | | ZA 200006008 A | 26-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 38486
- RU 43044
- US 6380223 B1
- US 6436986 B1
- US 6468975 B1
- US 20020147336 A1
- US 20020107235 A1
- US 20040014741 A1
- US 20040176595 A1
- WO 2004009017 A2
- WO 2004110385 A2
- WO 2004111015 A1
- US 20040266758 A1
- US 20040266831 A1
- WO 200116128 A1
- US 6274582 B1
- US 6642236 B1
- US 20060014758 A1
- US 6492520 B1
- US 6245769 B1
- US 5880135 A
- EP 0691128 B1
- WO 9911643 A
- US 20010025042 A1
- US 20030229091 A1
- WO 02089814 A1
- US 5057521 A
- JP 9071586 A
- WO 200176574 A2
- WO 2004046145 A1
- WO 2004014914 A1
- WO 2005118581 A1
- WO 2005118557 A2
- WO 2005118541 A2
- US 4617307 A
- US 4889861 A


### Non-patent literature cited in the description


- **MORGAN et al.** *J. Med. Chem.,* 2002, vol. 45, 2417-2424
- **PONT et al.** *Ann. Intern. Med,* 1982, vol. 97, 370-372
- **ENGELHARDT et al.** *Klin. Wochenschr.,* 1983, vol. 61, 373-375
- **VAN TYLE.** *Pharmacotherapy,* 1984, vol. 4, 343-373
- **SCHTEINGART ; CONN.** *J. Clin. Endocrinol. Metab.,* 1967, vol. 27, 1657-1666
- **WIPPLE et al.** *Steroids,* 1981, vol. 37, 673-679
- **LAMBERT et al.** *Mol. Cell. Endocrinol,* 1984, vol. 37, 115-120
- **LAMBERTS et al.** *J. Clin. Endocrinol. Metab.,* 1989, vol. 69, 896-901
- **LAMBERT et al.** *Ann. Clin. Biochem.,* 1986, vol. 23, 225-229
- **TOUITOU et al.** *J. Endocrinol.,* 1979, vol. 82, 87-94
- **WHIPPLE et al.** *Steroids,* 1981, vol. 37, 673-679
- **ULMSCHNEIDER et al.** *J. Med. Chem.,* 2005, vol. 48, 1563-1575
- **ANDREWS et al.** *J. Clin. Endocrinol. Metab.,* 2003, vol. 88, 285-291
- **LI.** *J. CLIN. ENDOCRINOL. METAB.,* 2004, vol. 53, 600-606
- **MORRIS et al.** *J. CLIN. ENDOCRINOL. METAB.,* 2004, vol. 53, 811-816
- **SAMPATH-KUMAR et al.** *J. CLIN. ENDOCRINOL. METAB.,* 1997, vol. 62, 195-199
- **COPPOLA et al.** *Metabolism,* 2005, vol. 48, 6696-6712
- **SCHWEIZER.** *Mol. Cell. Endocrinol.,* 2003, vol. 212, 41-49
- **CHEN et al.** *J. Med. Chem.,* 1997, vol. 40, 1749-1754
- **CHEN et al.** *J. Med. Chem.,* 2004, vol. 47, 4787-4798
- **DEMERS et al.** *the J. Clin. Endocrinol. Metab.,* 1990, vol. 70, 1162-1166
- **FIEBELER et al.** *Circulation,* 2005, vol. 111, 3087-3094